(19)

Europäisches
Patentamt
European
Patent Office
Office européen
des brevets

(11)     **EP 3 616 619 A1**

(12)                                    **EUROPEAN PATENT APPLICATION**
                                        published in accordance with Art. 153(4) EPC

(43) Date of publication:
     **04.03.2020 Bulletin 2020/10**

(51) Int Cl.:
     ***A61B 5/16*** *(2006.01)*        ***A61B 5/02*** *(2006.01)*

(21) Application number: **17929793.2**

(86) International application number:
     **PCT/RU2017/000791**

(22) Date of filing: **27.10.2017**

(87) International publication number:
     **WO 2019/083392 (02.05.2019 Gazette 2019/18)**

(84) Designated Contracting States:
     **AL AT BE BG CH CY CZ DE DK EE ES FI FR GB
     GR HR HU IE IS IT LI LT LU LV MC MK MT NL NO
     PL PT RO RS SE SI SK SM TR**
     Designated Extension States:
     **BA ME**
     Designated Validation States:
     **MA MD**

(71) Applicant: **Wehireai Inc.**
     **Redwood City, CA 94063 (US)**

(72) Inventor: **MIKHAJLOV, Igor' Valentinovich**
     **Moscow 119571 (RU)**

(74) Representative: **Papula Oy**
     **P.O. Box 981**
     **00101 Helsinki (FI)**

(54)     **METHOD OF PREPARING RECOMMENDATIONS FOR TAKING DECISIONS ON THE BASIS
         OF A COMPUTERIZED ASSESSMENT OF THE CAPABILITIES OF USERS**

(57)     The present technical solution in general relates to methods and systems of computer technology, in particular to automated computer methods and systems which help users and companies to take decisions on the basis of a computerized assessment of user capabilities. A method for preparing recommendations for taking decisions on the basis of a computerized assessment of the personality and capabilities of users, in which a user is given access to at least one computerized task for the execution thereof; emotions of the user are identified in real time during the execution of the at least one task by means of at least one video camera; data are obtained about the user's execution of the at least one task; at least one capability of the user is determined on the basis of data obtained after the at least one task is executed and the identified emotions of the user; recommendations for the user are formulated for taking decisions on the basis of the at least one capability determined in the preceding step. The technical effect is to raise the completeness, accuracy and speed of evaluating user capabilities.

Figure 4

EP 3 616 619 A1

## Description

BACKGROUND

This technical solution generally refers to the methods and systems of computer science, particularly to computer-automated methods and systems that assist users and companies in making decisions based on a computerized assessment of the personal qualities, interests, inclinations, talents and abilities of users as well as their mutual relations with the use of both artificial intelligence and emotion recognition.

PRIOR ART

Currently, recruiting suitable candidates for a position can be a daunting task for many companies. Generally, companies rely on intermediaries (external and internal recruiters) as well as on pre-selection and interviews conducted by these intermediaries in order to determine formal compliance with both the vacancy requirements and the company's corporate culture. However, the process of searching for new employees can be labor intensive, expensive and even ineffective in some cases. In addition, the search process may not take into account the full list of possible candidates, especially if there are too many applicants, and companies may incur both direct and indirect losses from the hiring and early dismissal of an unsuitable candidate. The recruitment may involve the distortion of information on the requirements for candidates, and the same description of the vacancy requirements can be interpreted differently by a hiring manager, a recruiter and a job-seeker. Additionally, a person can understand a great deal more information (verbal and non-verbal) in a face-to-face conversation rather than a distant one, but written requirements and CVs are the basis for preselection within the traditional approach. Thus, the screening out of candidates at the preliminary stage is subject to errors. Furthermore, errors may arise due to the intentional distortion of CVs by some candidates in an attempt to best meet the requirements of a vacancy as well as to increase their chances of getting an invitation to interview. When candidates who do not meet the vacancy requirements send in their CVs, this places an additional burden on the recruiters. An overflowing stack of CVs for vacancies leads to a superficial analysis and/or the use of automated CV ranking tools based on semantic similarity to the vacancy description, which reduces the quality of the pre-interview selection. At the same time, the interview may be subject to errors due to the interviewer's subconscious bias that arises from their own past life experiences. Thus, candidates who have more personal similarities with interviewers, and not necessarily those who would provide the highest measurable benefits for the company, can be selected. Recruiters and hiring managers must have sufficient skills to conduct both a qualitative in-depth interview and an objective analysis of a candidate for the position since the probability of errors increases where these are lacking. For example, the lack of the skill to recognize facial micro expressions does not allow the interviewer to determine when a candidate is hiding the true state of affairs and can cause them to give in to wishful thinking.

Similarly, in relations among people in general, as well as among colleagues within the framework of the boss-subordinate paradigm, the lack of this skill reduces the ability to effectively build compatible teams and provide recommendations on interpersonal relations.

On the other hand, determining a suitable career path based on one's skills can be a daunting task for job-seekers, and existing job search resources are often not tailored to an individual. A person's vision of a career path may be distorted by external circumstances, the promoted popularity of certain professions by the media, and the recommendations and opinions of his relatives and acquaintances rather than determined by his own deep understanding of his own individual development needs, abilities and preferences.

The situation is complicated by the rapid changes taking place in many industries and the emergence of new professions that did not previously exist as well as changes in the requirements for the existing professional qualifications. The need to maintain professional qualifications leads to the necessity of continuous training. The supply of educational services has substantially increased (including through the availability of online distance learning); therefore, it is difficult to get oriented about what will suit a particular individual at the moment. Taking into account personal goals, abilities and preferences, it is also difficult to assess what training might give the most effective result in terms of career and self-realization.

Employers, educational institutions, banks, insurance companies and other organizations are forced to focus on the average user metrics because of the lack of detailed data about personal traits and the individual abilities of existing and potential clients. The availability of such detailed information would allow for segmenting of clients more accurately as well as making more personalized offers of products and services.

In the prior art, there are automatic human resource management systems that allow for the solving of the aforementioned problems to some extent; however, the accuracy of such recommendation systems can be improved by taking into account a wider range of analyzed parameters, such as US Patent No. 6275812 B1, "Intelligent system for dynamic resource management," patentee: Lucent Technologies Inc., publication date: 14.10.2008. The system establishes a highly controlled approach to allocating members of an established, comprehensively cataloged workforce to equally well-cataloged projects. The detailed skills of each workforce member are presumed to be fully and uniformly assessed on skills templates. Each project is equally defined in terms of the detailed project requirements for project team members as reflected in the corresponding project template. The system matches the skill templates against the project templates.

A clear disadvantage of this technical solution is that additional skills are not taken into account, and skills that are not explicitly recognized by the system are ignored entirely. Clearly, the potentially valuable abilities of a workforce member who is not easily subject to strict categorization are also ignored. As a result, the preemptive conditions on the abilities of a person and those required in the project leads to a very rigid and exacting evaluation of candidates, which results in the loss of completeness as well as accuracy.

SUMMARY OF THE INVENTION

This technical solution is aimed at addressing the weaknesses that are inherent to solutions known from the prior art as well as to traditional recruitment methods.

A technical task or, in other words, a technical problem solved in this technical solution is the determination of a person's abilities on the basis of features that identify him clearly.

The technical result achieved in solving the aforementioned technical problem is to improve the completeness, accuracy and speed of assessing a user's abilities.

This technical result is achieved by implementing a method for the preparation of recommendations for decision-making that is based on the computerized assessment of a user's abilities, which implies granting the user access to at least one computerized task for a solution, real-time recognition of the user's emotions while solving at least one task in the presence of at least one video camera, obtaining data about at least one task solved by the user, determining at least one of the user's abilities based on both the data obtained after solving at least one task and on the user's recognized emotions and providing the user with recommendations for decision-making based on at least one ability determined in the previous step.

In some embodiments, a computerized task is a computational task, a video interview and/or a game.

In some embodiments, a biometric authentication is performed when a user is granted access to at least one computerized task for a solution.

In some embodiments, user identification is performed when recognizing a user's emotions.

In some embodiments, sensors of wearable devices are used to help in recognizing a user's emotions.

In some embodiments, technical means and oculography methods are used for recognizing a user's emotions and psychophysiological state.

In some embodiments, a user's emotions are recognized based on his speech by taking into account their rate of speech and/or tone.

In some embodiments, a convolutional neural network and/or a recurrent neural network is used to assist in recognizing a user's emotions.

In some embodiments, the data relating to the solution of a task completed by a user are the user action parameters that are fixed during the solution process.

In some embodiments, a user's ability score is determined by presetting weighting factors to the data relating to the solution of a task completed by the user, the fixed user's actions and/or recognized emotions.

In some embodiments, a recommendation that can be made for the user is a vacancy and/or a set of vacancies that may be of interest to the user.

BRIEF DESCRIPTION OF THE DRAWINGS

The features and advantages of this technical solution will become apparent both from the following detailed description and the attached drawings, which demonstrate the following:

Figure 1 shows a block diagram of an example for implementing a method and a scheme for preparing recommendations for decision making based on the computerized assessment of a user's abilities on the basis of the recognition of a user's emotions.

Figure 2 illustrates the Balloon Analogue Risk task. A virtual balloon is shown to a user in every task trial. The user can then click on one of the buttons to pump the balloon up or collect money. In the case of (A-D), the user receives a virtual monetary reward. At any time, the user can collect money and save the accumulated income (E). There is a nonzero probability of a balloon explosion when the user clicks on the "pump up" button. If the balloon explodes, the participant will not earn money for this trial (F).

Figure 3 shows an example of recognizable emotions of users with their distinctive features.

Figure 4 represents the conceptual architecture of the system for preparing recommendations for decision-making based on the computerized assessment of a user's abilities.

Figure 5 shows the recognition of a user's emotions by means of a video camera mounted on the device on which a task is performed by the user.

Figure 6 shows an embodiment when, for example, a poker game is played by a user instead of them doing a computerized task. The results and the sequence of decisions made by players can be analyzed by video analytics, which take into account the principles of neuropsychology.

Figure 7 shows the recognition of a user's emotions based on sensors of wearable devices.

Figure 8 shows an example of a user's data on the basis of which the user's abilities are determined on the data processing device.

DETAILED DESCRIPTION OF THE INVENTION

The concepts and definitions necessary for a detailed disclosure of the embodied technical solution will be described below.

In this technical solution, the personal characteristics, inclinations, talents and user preferences can be used similarly to their abilities.

The methods and systems implemented in this technical solution allow for determining the suitability of candidates aspiring to a certain position when it comes to the position requirements by assessing the candidates' behavior results that are obtained from one or more tasks or tests based on the principles of neurobiology and psychology, as shown in the source of information [3], including taking into account the emotional response of a person as evidenced by their facial expressions. The behavioral result of candidates can be compared to the employee model that is a typical variant of the employee for a particular position within a given company, taking into account the known effectiveness and/or personal qualities of the company's employees or what are typical for that market and/or an industry in general. Obviously, the concept of a typical candidate for a position involves some error, which is acceptable in certain embodiments. Many tasks based on the principles of various neurosciences can be presented as graphic games implemented in a mobile application or computer software or in a virtual and/or augmented reality interface that are designed for testing and measuring a wide range of emotional and cognitive features of the person who is playing those games. In addition, the emotional reactions of a candidate when performing a task or to an interlocutor during the game and/or interview can be analyzed with the use of an application or devices that analyze facial expressions, eye movement, gaze direction, body movement and/or biometric parameters. The use of neuroscience-based tasks, the emotional response of a user and the analysis of the results of these assignments and/or interviews allows companies to optimize the hiring and selection process by reducing both the human factor impact of the representative of the hiring party and the shortcomings of pre-selection based on limited information in the candidate's CV and/or social profile. In addition to the fact that this information is a useful recruiting tool for companies, the systems and methods described herein can help people in building teams, analyzing interpersonal relations and making recommendations on people's compatibility as well as planning careers and identifying talents. The implemented systems and methods can identify the strengths and weaknesses of a test subject by using the tests that measure a wide range of emotional and cognitive features. This information can then be applied to recommendations for developing a person's own work skills or interpersonal skills as well as for choosing the preferred professions and companies with a suitable corporate culture. In addition, the systems and methods described herein can help companies to segment clients more accurately and determine whether a potential client matches the target segment. By identifying an additional range of personality traits, companies and individual entrepreneurs can expand the decision-making models that they use to rank clients and make decisions, including the cost of products and services, among others. For example, responsibility, honesty, diligence, a propensity to avoid risks and other individual characteristics revealed in potential or existing clients can help to identify more trustworthy clients who will generate more income, fewer losses etc. Also, recommendation systems of electronic stores or other systems of companies and individual entrepreneurs that provide products and services can be specified based on the aforementioned identified individual traits of a person. This can contribute to a more accurate prediction of a buyer's interests; an understanding of what might better influence the weighted or spontaneous decision on making a purchase; what type of targeted advertising is the most effective for a particular client as well as in what form it should be displayed, how many times it should be shown, when it should be displayed etc.

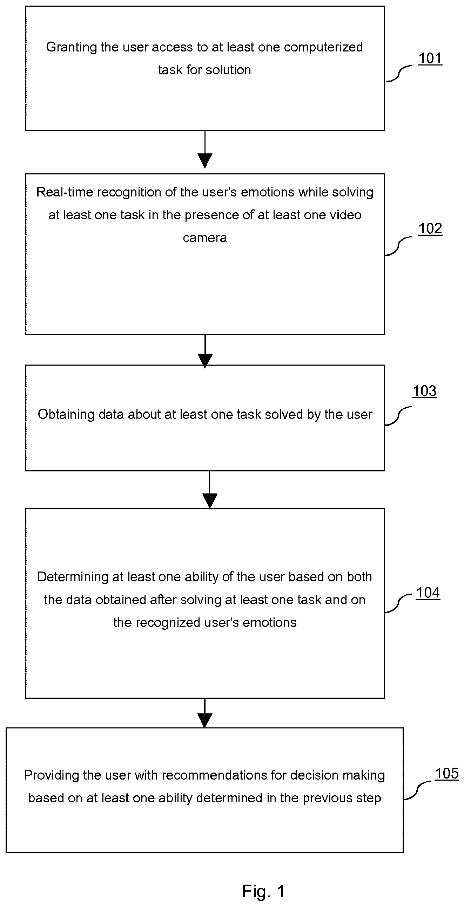

The following steps are carried out according to an embodiment of the method used for the preparation of recommendations for decision-making based on the computerized assessment of both a user's personality and abilities, shown in Figure 1.

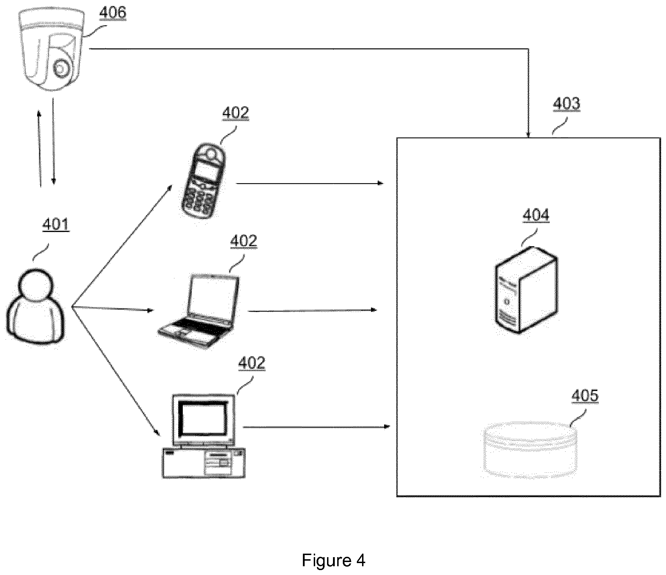

**Step 101:** providing the user **401** with access to at least one computerized task for solution on the device **402,** as shown in Figure 4.

In some embodiments of the present invention, a computerized task is a computational task or a computer game, including but not limited to a virtual and/or augmented reality interface. In other embodiments, the task is a video interview with an additional application that analyzes video and/or audio and/or recognizes speech, kinetic and/or biometric parameters of a user, assesses people's compatibility and their reaction to the parameters of goods and services as well as analyzes emotions, stress level, and any discrepancies between subconscious reactions and the created image.

Video interviews can be carried out in the "human-human" or "human-robot" format of interaction. In other embodiments, within the video interview, the user **401** is provided with questions on the graphical interface of the device **402,** and the user's answers to these questions are recorded.

In the present technical solution, the user **401** may be a student or a graduate student planning his career and studies, including continuing education and professional development and learning through self-study as well as through training programs and institutions. In other embodiments, the user **401** is a person searching for a job. The user **401** can answer quizzes or finish computer games to which he is granted access or he can undergo a video interview, after which the system can create a user profile based on his specific abilities and/or information about him that was entered by the user. In other embodiments, it may be a buyer of goods and services or people whose individual or average characteristics serve as the basis for conclusions made by recommendation systems.

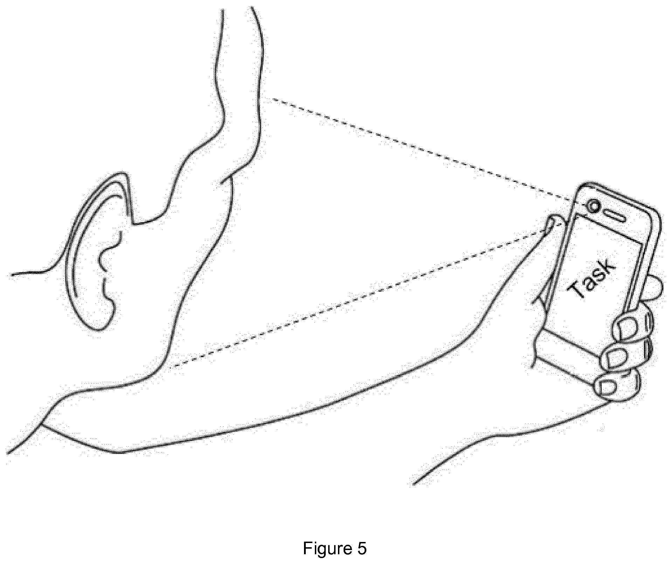

The user 401 creates a login and a password and fills out personal information, after which he authenticates and takes up the task, thereby gaining access to the assignment. In other embodiments, the user 401 receives a URL link to an Internet resource where the computerized tasks are located. Additionally, authentication can be performed by using a biometric authentication, such as by fingerprint authentication, iris recognition, facial geometry, facial thermogram, retina of the eye, voice, other biometric parameters etc., as shown in Figure 5. Then the user 401 can solve the computerized tasks of the system using either a portable or stationary computing device 402, such as a personal computer, laptop, mobile or wearable device or tablet. In some embodiments, the user 401 is then provided with recommendations regarding various occupations, professions and industry branches based on the skills and features of the user 401 that were either determined by the system or entered by him. Consulting, education, healthcare, marketing, retail, entertainment, consumer goods, entrepreneurship, technology, hedge funds, investment management, investment banking, private capital, product development, product management and more may be recommended as examples of suitable industries.

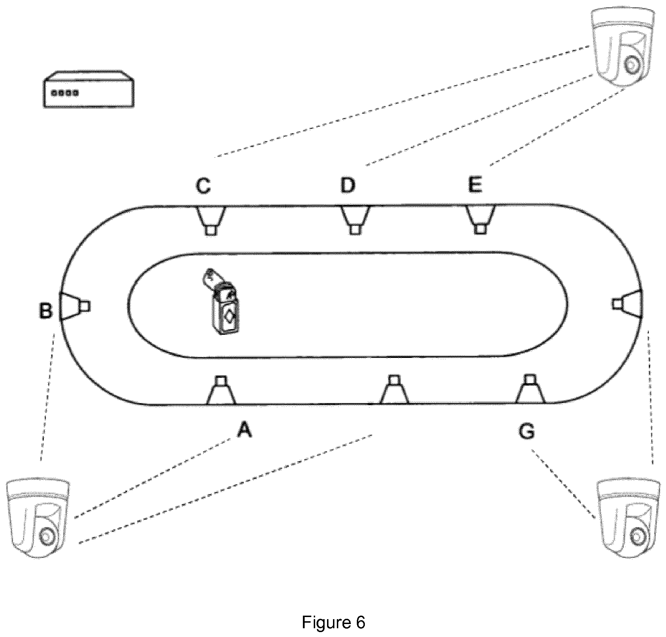

In some embodiments of the present technical solution, such as a poker game or a strategic game instead of a computerized task, the results of the game as well as the sequence of the decisions made by players can be analyzed by video analytics by taking into account the principles of neuropsychology, as shown in Figure 6.

According to various embodiments of the invention, there are several authentication factors, such as a two-factor authentication that can be used to provide users with access to accounts for accomplishing the tasks. For example, in addition to providing his login and password for accessing the account, the user 401 may also be invited to send a confirmation containing a verification code from a second device, such as the user's mobile phone, in order to gain access. Access to an account may be granted on the system server only after verifying the information relating to the second factor, including but not limited to the user name, password and mobile number. In other embodiments, another biometric identifier may be used as an additional authentication factor. For someone to gain unauthorized access to an account, this person would need to not only know the user's login and password but also the user's mobile number, which, for obvious reasons, will make it difficult for the intruder to gain access to the system. The intruder would need to be able to send and receive text messages from the victim's phone.

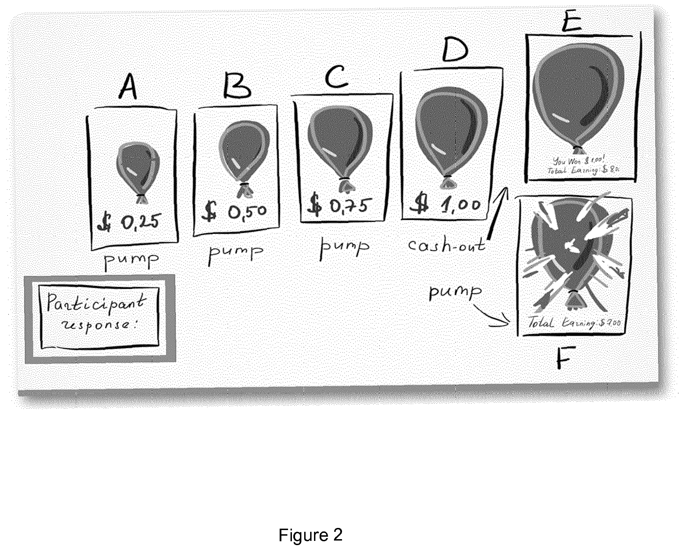

The tasks selected on the basis of various neurosciences or cognitive psychology applications can be used as computerized tasks for a user to determine a clear correlation between the result of the completed task and their own abilities. The examples of such tasks may be computational tasks of the prior art [6], such as Analogical Reasoning, Balloon Analogue Risk Task, Choice Task, Dictator Task, Digit Span, EEfRT, Facial Affect Task, Finger Tapping, Future Discounting, Flanker Task, Go/No-Go, Mind in the Eyes, N-Back, Pattern Recognition, Reward Learning Task, Tower of London and Trust Task, among others. Every computational task or multiple tasks done together can measure the features of the user that reflect his abilities. For example, the Balloon Analogue Risk task assesses the user's propensity for risk (Figure 2), and the Flanker Task assesses the tendency to evade answers that are inappropriate for the user in a particular context.

All user information, subsequently identified as a user's abilities, as well as automated recommendations prepared specifically for him or her, can be stored on the computer storage medium 405. Computer storage media include RAM, ROM, an erasable programmable read-only memory device (EPROM), an electrically erasable programmable read-only memory device (EEPROM), flash memory or other solid-state memory technology, compact disc read-only memory (CD-ROM), a digital versatile disk (DVD) or other optical storage devices, magnetic cassettes, magnetic tapes, magnetic disks or other magnetic storage devices, cloud storages or any other medium that can be used to store necessary information and that can be accessed by the user.

Step 102: Real-time recognition of the user's emotions while solving at least one task in the presence of at least one video camera

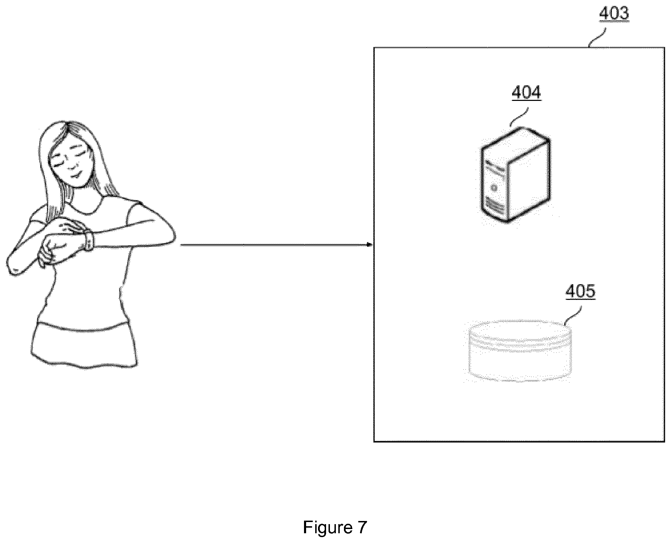

In other embodiments, the sensors of wearable devices are used to help in recognizing a user's emotions, as shown in Figure 7. For example, a user's pulse will speed up when he experiences anger or anxiety, which is recorded by the wearable device sensor and thus affects the determination of the user's abilities.

Real-time recognition of both the user's face and emotions that appear on the face by means of the video camera 406 takes place during performance of the task to which access was granted, as shown in Figure 5.

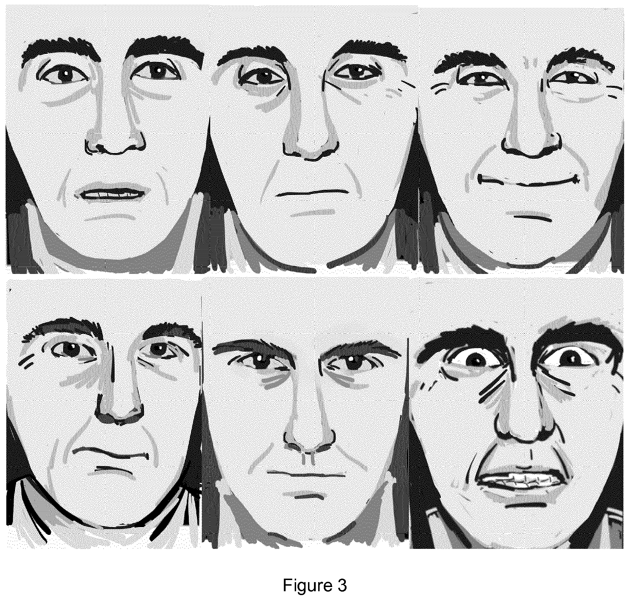

The embodiments described herein allow for detection and recognition of the images of persons and a wide range of their positions in front of the video camera 406, exposure to light, degree of user aging, his race etc. The traditional methods for recognizing faces known from the prior art described in the work [4] may be used in the present technical solution as well. The modern methods of face recognition offer real-time solutions that provide high recognition rates, including through the analysis of a 3D face model. In some embodiments, there may be pre-arranged datasets that are used for automatic recognition of emotions by an artificial neural network, such as the extended Cohn-Kanade (CK +) data set, the MMI face expression database and the Toronto faces data set (TFD). The Ekman and Friesen method [5] can be used for determining emotions shown through a user's facial expressions, which allows for encoding the facial movements (FACS), where anatomical movements on the face are described by a set of action units that have some associated muscular base. The method that is used classifies various facial expressions and emotions by tracking facial movements and measuring a number of those movements. According to one of the method embodiments, when recognizing emotions, a static 3D face model is created and/or a 3D face model is dynamically analyzed. The targeted facial features, such as the corners of the eyes and mouth, are selected in the first frame of the sequence of images obtained from the video camera. The next step involves the analysis of any changes in the selected facial features. Classification and recording of emotions are carried out based on these changes, which are approximately shown in Figure 3.

Convolutional neural networks (CNN) in this technical solution can be used to recognize a user's facial expressions and the emotions they signify. The input of an artificial neural network is a set of human facial expressions obtained from the video camera **406** or from the storage medium **405** of the system **403,** after which a convolutional neural network is used to recognize the user's facial expressions or emotions (i.e., classify the obtained facial expressions on the basis of the trained neural network). The identified emotions can be anger, happiness, fear, sadness, joy, disgust, surprise and neutrality, among others, as well as their combinations (Figure 3). In some embodiments, a convolutional neural network (CNN) is used in conjunction with a recurrent neural network (RNN).

In some embodiments of the invention, other methods, such as Bayesian networks, multilayer neural networks, hidden Markov models, decision trees, SVM, kNN and more, may be used to classify emotions.

Additionally, in the present invention, it is also possible to recognize emotions based on a user's speech by taking into account its speed, tone etc. For example, when a user starts feeling anxiety, his speech may accelerate. Different people speak at different speeds in a relaxed state. In addition, in some languages, the speech is more measured, while in others it is chopped and fast.

The mobile phone **402,** tablet or computer on which a user performs a computerized task may also contain one or several video cameras **406** that are either built in or external, and one or several of those cameras may be located on the same side as the screen. In the alternative embodiment, the video camera may be either located on the other side of the screen or may be an external camera.

The tasks are solved directly through a graphical user interface that may comprise a display and/or a connector for an external display or a data projector as well as a keyboard or other applicable means of control (for example, a touch screen or a microphone for voice control or certain keys, buttons, regulators or switches) that is configured to provide the user of the device both with data visualization and a means for controlling the device. In addition, the user device **402** may contain a transceiver that comprises a radio beacon that includes a wireless transceiver, such as a WLAN or GSM/UMTS/LTE transceiver, for general connection with other devices, network infrastructure and/or other means of wireless or wired data transfer, such as one or more wired interfaces (for example, HDMI or USB) for connection with other devices, such as terminal devices, peripherals that include external sensors or a network infrastructure. It is obvious that for a person skilled in the art, the device may comprise a variety of additional functional and/or structural elements to provide the user with solutions to the tasks, and this detailed disclosure should not be construed as the restricting presence of additional elements by any means.

In other embodiments, the tasks are solved by using a voice assistant, such as Alexa, Cortana or Siri.

Additionally, in some embodiments, a person is recognized and identified by the video camera **406** based on a previously acquired photo or a set of user photos.

**Step 103:** Obtaining data about at least one task solved by the user

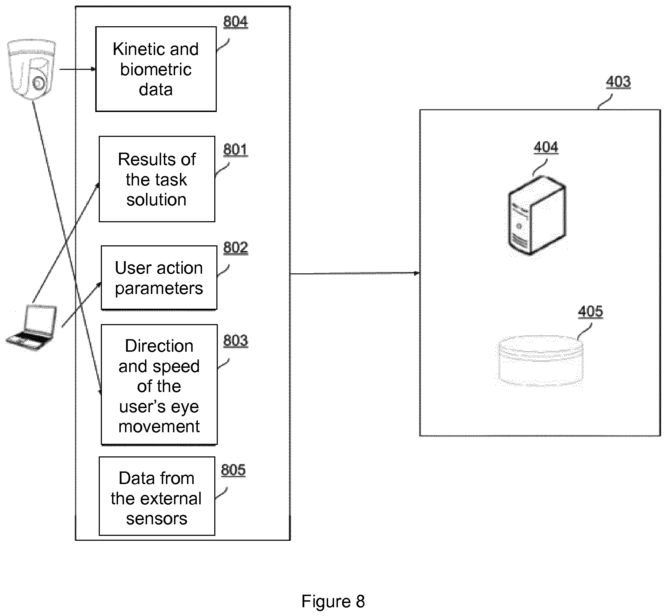

The data about at least one task solved by the user are the results **801** of the finished computer game, solved computing task or passed video interview, depending on the task type, as shown in Figure 8. In addition, the data may include the user action parameters **802** that were fixed during the solving of the task by the user. These parameters can be the coordinates of the place of pressing the screen with one or more fingers, the force of pressing, the frequency of clicks, the places of clicks by the computer mouse cursor, stylus, its analogs or the user's actions on the keyboard. Unlike video games, the measure of success in an educational game, which has the purpose of determining the user's abilities, is not just how many levels were passed by the user but the intensity of the user's game playing. For example, the time during which the user does not perform any actions may provide information about the task fulfillment and indicates the user's mental activity on the task. Therefore, it is possible to draw some conclusions about his abilities. Additionally, the direction and speed **803** of the user's eye movement can be statically or dynamically determined by the video camera **406.** In the case of the use of mobile and/or wearable devices, a change in the device position in the space can be registered by using an integrated gyroscope as well as other kinetic and biometric parameters **804** registered by these devices.

For example, user-related data, such as physiological data obtained through determining a user's state by monitoring heart rate, body temperature, blood pressure or perspiration, can be collected in some embodiments. In other embodiments, the data relating to a device on which the user solves tasks, such as device status information and/or information about the memory contents, can be collected with the user's consent. The environmental data **806,** such as information about temperature or atmospheric pressure from external sensors, can also be collected. Different types of input data can also be used in the same process of determining the user's abilities.

The aforementioned data come to the data processing device **404** of the system **403** from the user device **402** on which the task is performed as well as from other external sensors, as shown in Figure 8.

**Step 104:** Determining at least one user's ability based on both the data obtained after solving at least one task and on the user's recognized emotions

The prediction of personal characteristics as well as the evaluation of personal qualities, interests, inclinations, talents or abilities of the user takes place based on both the data obtained after solving at least one task and on the recognized emotions of the user.

All of the aforementioned data, including but not limited to the data on the user's interaction with tests, tasks, information about emotions, micro mimics and facial expressions during task execution, are used as input parameters for algorithms based on machine learning methods. These algorithms can include both supervised learning methods, such as regression analysis, discriminant analysis, generalized linear models, decision trees, support vector method and neural networks and unsupervised learning methods that include cluster analysis, the method of principal components and self-organizing maps. The result of the algorithm is a classification of users for compliance with the requirements necessary to fill a particular vacancy as well as the determination of the level of cognitive abilities and emotional intelligence of the user.

In addition, a forecast can be made of the user's compliance with the company's corporate culture, the likelihood of achieving the required results at the relevant position at the company, the propensity for fraud and more as well as an inclination to certain professions or greater efficiency in one or another kind of activity.

Every assignment corresponds to a set of abilities that correlate with the given task type. The search for a correspondence among the metrics obtained during task execution by the user (time for solution, time for thinking, speed and more) as well as a set of the user's observed emotions and abilities is carried out.

In the simplest form, some abilities can be determined as follows:

$$P_i = \alpha \times \frac{N_{current}}{N_{max} \times f\left(t_{current}\right)},$$

where $N_{current}$ is the number of points obtained while completing the task, $N_{max}$ is the maximum number of points that can be obtained for the task (taking into account the number of attempts), $f\left(t_{current}\right)$ is the function depending on the duration of the task execution and $\alpha$ is the scaling constant for each task type.

In some embodiments, a user's ability score is determined by presetting weighting factors both for the data relating to the solution of a task by the user and the recognized emotions. The data relating to the solution of a task are preliminarily converted into a numerical value, and the recognized emotions can be transformed either into an ordinal or interval scale.

For example, the ability indicator may be computed as an affine combination of the data based on the user's task solving, in numerical equivalent, using the recognized emotions of the user:

$$P_i = R \times \alpha + S \times (1 - \alpha),$$

where $R$ is the key indicator relating to the task solving, $S$ is the data relating to the recognized emotions and the weighting factor $\alpha$ is a real number with a value between 0 and 1. The weighting factor $\alpha$ may be set in any appropriate way. In some cases, it may be set on the basis of the relative importance of the data related to the recognized emotions S. As described above, a high data factor may indicate the relative importance of the data that may be possessed by the user. Consequently, the weighting factor $\alpha$ may be set at (or may be set based on) the level that determines the relative importance of the data.

Every ability can be expressed as a numerical value or in percentage terms. According to various scoring methods, the time and total number of points obtained, from 0 to 100, while completing the task can be taken into account in a manner similar to other standard scales of intelligence, such as Wexler or Raven tests.

**Step 105:** Providing the user with recommendations for decision-making based on at least one ability determined in the previous step

At this stage, after the user's abilities have been determined, he is provided with information about his abilities. For example, the system can provide the user with a score for every ability for one or more attempts at a task.

To give another example, the system for determining a user's abilities may recommend one or more vacancies and/or types of vacancies based on the defined abilities that may be of interest to the user as well as provide the user with any appropriate information. For example, the user may be offered one or more additional tasks if his ability indicator value exceeds a certain threshold for that ability.

The user may be provided with information on what value and abilities are needed for a particular job in comparison to the abilities of other users who applied for the same job. Alternatively, or in addition, the user may be provided with scores for the abilities of other users (in an anonymized form) whose assessments were defined for each task. In some embodiments, if the ability determination system has received the user's permission to publish information about him, this data can be shown to other users in various forms. For example, it could be a position on the user's results chart regarding the variability and/or distribution of other users' results.

In addition, the claimed technical solution can be implemented as a method, a device or a system using standard software and/or engineering methods for developing software, firmware, hardware or any combination thereof for controlling a computer in order to implement the disclosed invention.

Some parts of this invention are presented in terms of algorithms and other representations of operations with data bits or binary digital signals in a computer memory. It is appreciated that definitions or conclusions mentioned in this description can be implemented with the use of artificial intelligence techniques. More specifically, the terms "processing," "calculation," "determination," "establishment," "analysis," "identification," "verification" etc., may relate to the operations and/or processes of a computer, computing platform, computer system or another electronic device that manipulates and/or transforms data represented as physical (electronic) quantities within the computer registers and/or memory devices into other data similarly represented as physical quantities within registers and/or storage devices of a computer or another storage medium that can store instructions for executing the aforementioned methods, operations and/or processes.

EXAMPLES OF THE EMBODIMENT

The person skilled in the art can clearly see from below-mentioned examples the technical solution implementation and embodiment shows that the solution is capable of industrial applications.

**Example 01:** When students and recent college graduates with little work experience, as well as people changing a profession, are offered a job, the information on their previous work experience, CVs and recommendations may not be enough to preliminarily assess the subject matter competencies required in a new position. In addition, the information contained in a worker's letters of reference and recommendations may not be enough when using sub-contract work (on a contract and/or voluntary basis such as with the use of infocommunications technologies like crowdsourcing and/or crowdworking).

For example, in the sales offices of a company, 13,000 workers (65% out of 20,000 hired per year) quit within a short time. The company spends a great deal of money on recruitment and training (roughly $250 per hire); however, the churn rate of such employees is 10 times higher than the churn rate of employees from conventional offices (the headquarters). Moreover, 3,250 of employees (25% out of 13,000 who are offered a job per year) are mistakenly invited. As a result, employees resign from their jobs. The company loses $812,500 a year solely on the recruitment and training of these 3,250 employees. The amount increases several-fold when losses from all departed employees are calculated, both with sales inefficiency and reduction of client loyalty due to poor service.

This presents the challenge of both differentiating substantive candidates inclined to a particular profession and determining the most productive position for them by using a mobile gaming application as well as identifying a user's abilities via a virtual and/or augmented reality interface. At the same time, it is necessary to reduce errors when determining the probability of the presence or absence of the aforementioned qualities as well as increase resistance to falsification of test results.

Before a new candidate for the position of a sales manager is admitted for an interview, he receives either an activation code or an individual URL link to the described technical solution, which determines his abilities as well as automatically predicts how promising he will be as a salesperson (in this company or in the profession in general). At the same time, the game is arranged so that it is not obvious to a candidate how the measurement of his skills and abilities is taking place, so the candidate cannot falsify the results, including asking another player to play instead.

Eventually, a company pre-qualifies candidates according to their abilities and prioritizes the highest-rated candidates for interviewing.

The more qualitative selection of candidates excludes entry to the profession for those who are not inclined to this type of work. As a result, the outflow of employees is reduced to the average market rate, which reduces the losses that stem from mistakenly recruited employees. Improving the quality of selection according to a candidate's labor productivity forecast leads to an increase in both client loyalty and in a specific volume of sales.

**Example 02:** When human resource managers receive more responses to vacancies than they can effectively process, they have to reduce the average amount of time spent to analyze a CV and/or use automatic tools to analyze CVs for semantic similarity to the vacancy description. These and other methods for the automated analyzing of candidates' CVs, as well as their profiles and activity on social networks, help to reduce time for analysis, but they also reduce the quality of the search. In addition to the fact that a CV reflects very limited information about a candidate, it may be self-optimized by candidates for such analytical systems, which may create an advantage for less suitable candidates. Not all candidates are able to correctly draw up CVs or effectively use techniques for their optimization. Additionally, both CVs and information about the candidates are self-edited in social networks with the goal of creating the desired self-image, which does not necessarily coincide with the real one.

Let's assume a hiring manager, a recruiting department head in a large company or an agency acting on their behalf publishes thousands of job descriptions, either on their own or on external sites. Candidates respond to these even if they do not necessarily meet the requirements of the vacancies. As a result, the human resource manager spends a great deal of time screening out more than 80% of the CVs that are unsuitable. Because of time constraints, the average time spent on the assessment of a CV may be less than 10 seconds. As a result, a CV of a more suitable candidate may be inadvertently discarded, while close attention might be paid to a less suitable CV.

This raises the challenge of the effective screening of candidates based on non-editable information (containing real data about the candidates, not the desired reality image). It is necessary to ensure that only those who meet the qualification requirements and are the most suitable according to their inclinations, character and mindset can respond to vacancies. At the same time, the preliminary qualifying testing for compliance with these requirements should take place automatically, without the direct involvement of a company's representatives, in order to upscale the search coverage for candidates. It is necessary to carry out a reliable control over a user's authentication so that the results of testing cannot be falsified by transferring the tasks to another person.

Candidates who wish to work in a particular company will receive an individual URL link or an activation code to the mobile game that automatically predicts whether the candidate meets both the vacancy requirements and the ideal candidate profile by determining his abilities. At the same time, the game is arranged so that it is not obvious for a candidate how the measurement of his skills and abilities is taking place, so the candidate cannot falsify the results (including asking another person to play instead).

As a result, a company pre-qualifies candidates according to their abilities. Afterwards, they receive access to information about the vacancies they fit the best. Alternatively, a company may not publish vacancies in the public domain, may do it partially or may consider CVs of pre-qualified candidates first in order to completely exclude the responses of unsuitable candidates.

As a result, more than 80% of unsuitable CVs are automatically sorted without significant losses in the quality of analysis, which reduces the workload of a human resource manager. Since a company's human resources are not spent on such pre-sorting, the process can be unlimitedly upscaled.

In addition to information related to a candidate based both on his CV and social network account, which is traditionally used for the analysis, a manager also obtains extra information related to personal qualities, interests, inclinations, talents and abilities of the candidate. This in turn improves the selection of candidates and contributes to better employee satisfaction with their jobs that correspond to their abilities and preferences, increasing productivity and reducing the outflow of personnel after recruitment.

**Example 03:** When assessing personal compatibility as well as building teams, people have to focus on their ability to make judgments about other people. At the same time, these judgments can be distorted by a biased opinion that was either consciously or subconsciously gained on the basis of both previous life experience and imposed stereotypes. Furthermore, not everyone has undergone special training for making such judgments efficiently.

Let's assume a human resource manager, a hiring manager, a buyer of a product or service who is being convinced by a salesperson and a person who wants to better understand the relationship with another person takes part in an interview, a business meeting or another conversation. The interaction of these people during a direct face-to-face conversation provides a lot of useful information. Particularly, micro reactions and micro expressions that occur at a subconscious level are manifested in the movements of the eyes, eyelids and corners of the mouth as well as perspiring, gestures and more. These subconscious and uncontrolled reactions can be either short term in nature or disguised as deliberately demonstrative reactions in order to create the desired impression. Additionally, emotional reactions can be traced by changing voice tone and volume, speech frequency, swallowing, face color, swelling, manifestation of veins and more.

The types of emotions experienced by an interlocutor, as well as discrepancies between visually recognizable subconscious and conscious reactions, emotions in the voice and the meaning of what was said, provide additional information for decision making. However, not every person has enough experience, qualifications and abilities to detect, recognize and properly classify both these emotions and their discrepancies.

This presents the challenge of efficiently recognizing and registering an interlocutor's emotions within the context and taking into account discrepancies between the content of speech, emotions in the voice and conscious and subconscious emotions displayed externally. Meanwhile, it has to be done automatically, both in the presence of a person and remotely (via a video link).

Those who wish to conduct an interview, a workshop or another conversation are offered to hold a private meeting, either in the presence of a video camera or via a video call through a mobile device or a computer with a built-in video camera. In the latter case, a URL link or an activation code is sent to all of the participants, and this launches a video session. Each interlocutor may either consent or refuse to recognize and register his emotions during this session and video recording. If at least one of the participants gives his consent, the data is recorded during this video session according to the permission received.

In some embodiments, during a meeting, a training or a game session (including desktop or business games) involving several people, either in one room or remotely connected from different facilities, actions and reactions of at least one of the participants, his emotions and reactions in relation to other participants and their actions at the meeting are video recorded using at least one external or built-in camera. In addition, biometric, kinetic and environmental parameters may also be recorded.

As a result, at the very least, an organizer of a video call/video recording (for example, an HR manager of a company who organized a candidate's interview with a hiring manager as well as with a potential future manager) obtains extra information: the aforementioned reactions of at least one interlocutor to another, the words he uttered and the tone of his voice as well as the aforementioned emotions during his own statements and other things during the video conference.

The organizer of a video call then obtains information about at least one interlocutor: about his stress level, personal affectations, manipulation (flattery, lies, psychological pressure etc.), degree of self-confidence and confidence in what was said as well as any conscious and subconscious reactions of a single interlocutor to another one (or to a group of interlocutors in the case of interaction by several participants). This includes what was said as well as any discrepancies between what was said (text) by others, what was heard (speech) from others and any visual reactions of other interlocutors seen in others that were perceived by this interlocutor. Thus, even in the absence of a record relating to an interlocutor's reaction, it is possible to draw conclusions about him based on the mutual reaction of other interlocutors with respect to him, his emotions, what was uttered by him and what was noticed about him during a conversation.

Eventually, the extra information obtained allows for a more balanced decision to be made about the personal compatibility of people or the building of teams.

**Example 04:** A requirement for increased accuracy of the analysis arises when such accuracy is needed to identity personal parameters as well as classify a user's actions, especially in a situation with an increased risk of incorrect decision-making, such as a high-cost error.

This raises the challenge of an in-depth analysis of personal characteristics as well as a more thorough assessment of the personal qualities, interests, inclinations, talents or abilities of a user while taking into account an expanded set of video, audio, kinetic or biometric information about the user or environmental parameters that were collected and analyzed. This applies to both the time of testing or a number of measurements with either time lags between them or continuously as well as individual research or group research based on interaction with other people for whom similar information may be collected.

Let's assume a candidate is considered for a vacancy of a financially responsible person, such as a cashier, an auditor or a financial director, and the vacancy requires access to particularly sensitive information or critical processes. In this instance, it is necessary to carry out a more thorough examination of the candidate, employee, contractor or others for compliance with the increased requirements. Examples of such requirements may include honesty in disclosing information, no propensity for fraud, a desire to comply with rules and obligations, resistance to manipulation and threats, self-control in extraordinary situations and more.

People who have granted their permission to conduct such research are offered to participate either in the presence of a video camera or via a video call through a mobile device or a computer with a built-in video camera. In the latter case, either a URL link or an activation code that launches a video session will be sent to all participants. In addition, the pulse rate, as well as other biometric and kinetic parameters, is read through a wearable device. Registration and recognition of information about the user's emotions and his reactions, including physical reactions, during the given tasks are carried out during the research sessions.

As a result, at the very least, an organizer of the research obtains information about at least one interlocutor concerning his stress level, personal affectations, manipulation (flattery, lies, psychological pressure etc.), degree of self-confidence and confidence in what was said as well as his conscious and subconscious reactions of a single interlocutor to another one (or to a group of interlocutors in the case of interaction by several participants). This includes what was said as well as any discrepancies between what was said (text) by others, what was heard (speech) from others and any visual reactions of other interlocutors seen in others that were perceived by this interlocutor, if the research included interaction with other people. Additionally, the extent to which a test subject manifests honesty in disclosing information, no propensity for fraud, desire to comply with rules and obligations, resistance to manipulation and threats, self-control in extraordinary situations and more can be forecasted depending on the purpose of the analysis.

This information about a test subject in the form of a single research project, several studies or continuous research within a given time allows a human resource manager, a hiring manager or an agent acting on their behalf to make more balanced decisions by taking into account the additional information obtained.

**Example 05:** When using an automated user authorization, there may be doubts as to whether it was this user who performed the authorization as well as whether he performed actions in the system after authorization. The types of usage may be different and are not limited to the example described below.

Let's assume a human resource manager sent a URL link or an activation code to a candidate so that he could take a test in an application, on a website or in a program on a computer. In addition, if the testing is performed remotely and not under the supervision of a trustee or an HR manager who can verify the compliance with the rules, this may raise doubts as to whether the candidate could transfer his credentials or otherwise transfer control to another user who could pass the test for him.

This raises the challenge of conducting an efficient authorization of a user when logging in to the system as well as during a session within the system in order to testify that the user is the actual author of these actions (logging in to the system as well as manipulating it) and to overcome the existing restrictions of authorization systems, such as to correctly distinguish twins from each other or distinguish people in face masks that exactly copy users' faces.

Multiple personal characteristics of a user are registered both during authorization in the system and during one or several manipulations with it or continuously. This includes recognition of facial features and types of emotional reactions during task execution as well as the quality of the voice, its tone, emotions in the voice, speech semantics and more. The collected comprehensive "digital emotional imprint" that characterizes a person is compared to the one who registered earlier. Additionally, a user may be asked to undergo the same comprehensive authorization procedure in the presence of an individual who needs to personally ensure that the user who performed the actions in the system is the same person he is communicating with.

For example, before signing an employment contract or during other stages of a recruiting process, an HR manager, a hiring manager or an agent acting on their behalf may ask a user to re-enter the authorization in his presence. Thus, he will verify that the user who performed the actions in the system is the same person. Since such authorization was carried out once, several times or continuously while using the system, he can then have appropriate assurance that it was the user who executed these actions at those times and that they did not transfer control to another person. As a result, losses from forgery of this kind are reduced.

**Example 06:** The existing methods of testing may be less effective when it is necessary to monitor progress in the improvement of users' skills or abilities, including through either long or relatively short periods of time. For example, if after completing a task, a test subject could figure out or guess what the test measures as well as how this measurement is carried out, this could give him an advantage when redoing the task. This knowledge could be used to change a strategy or tactic during the task in order to get a better result. Another example is a test subject trained to effectively solve tasks of a certain type by frequent repetition; however, after a long period of time, he showed a different result when solving the same task. There are other examples related to the sustainability of test results owing to the influence of knowledge, test methodology, dependence on frequency and the intensity of task repetition over time, as well as other reasons.

This presents the challenge of effectively conducting a test that is resilient to the frequency and intensity of repetitions and identifies changes in a user's personal characteristics, personal qualities, interests, inclinations, talents or abilities fixed over a long-term period (at subconscious level) as well as distinguishes them from non-fixed short-term changes (performed consciously), such as taking into account recently comprehended case studies.

Let's assume a training and development department head of a company needs to evaluate the effectiveness of ongoing training programs. On the one hand, the department head must both manage the portfolio of programs of various contractors and weed out inefficient trainings. On the other hand, he must determine which employees show the greatest increase in competencies based on the results of these trainings, for example, in order to make a decision on their retention by the company and further career development.

Employees who wish to attend one or several training programs receive an individual URL link or an activation code to at least one mobile game and/or another task that automatically evaluates one or more parameters: personal characteristics, personal qualities, interests, inclinations, talents or abilities. Furthermore, this user may be required to take this test repeatedly, with either longer or shorter time intervals. Since emotions (either conscious or subconscious reactions) are also taken into account during game playing or task solving, the obtained data relating to test results over time will allow for the prediction of the degree of change in personal characteristics, personal qualities, interests, inclinations, talents or abilities as well as how thoroughly these changes are fixed.

As a result, at the very least, the training and development department head of a company will obtain information about the actual effectiveness of ongoing training programs. Unlike the ability of employees to correctly answer questions that follow the appropriate training, it is evaluated whether the received practices were adopted, forgotten or ignored or became a part of active practice. In addition, this department head obtains a tool for the comparison of individual learning results of different participants, both on average and by individual parameters, as well as a possibility to consider trends both with a test subject and selectively with various combinations of groups. The latter can affect the decision to build teams for effective joint training, both on site and remote.

According to one of the embodiments of the present invention, it is possible to conduct individual or group training directly in the application where the testing is performed, including conducting it simultaneously or otherwise by combining it with the testing, such as via a mobile application.

**Example 07:** Determining a suitable career path can be a daunting task for job-seekers, and existing job search resources are often not tailored to an individual. A person's vision of a career path may be distorted by external circumstances, the promoted popularity of certain professions by the media and recommendations and opinions of relatives or acquaintances rather than determined by his own deep understanding of his individual development needs, abilities and preferences. The situation is complicated by the rapid changes in many industries and emergence of new professions that did not exist before as well as changes in the requirements for the existing professional qualifications. The number of times people change jobs during their lifetime is also increasing, and the need to maintain professional qualifications leads to the necessity of continuous training. The supply of educational services has substantially increased. Therefore, taking into account personal goals, abilities and preferences, it is difficult to get oriented about what will suit a particular individual at the moment. It is also difficult to assess what training might give the most effective result in terms of career and self-realization.

This raises the challenge of how to segment clients (up to segments consisting of one client) and how to make more personalized offers of products and services as well as how to provide a user with relevant recommendations on career development, choice of training programs and other products and services while taking into account the personal characteristics, assessments of personal qualities, interests, inclinations, talents or abilities of the user.

Let's assume a school or university student has not yet determined his preferences for a future profession or a professionally esteemed employee with working experience is either thinking about changing careers or wants to find a new vocation in an area where his abilities and interests will be more pronounced, which will allow him to realize his own potential effectively. At the same time, he wants to find a job that demands staff individuality rather than having to adapt himself to an alien corporate culture or other stereotyped behavior.

A person downloads an application that defines a psychological profile in the form of a game with a wide range of personal parameters. Meanwhile, the game is arranged so that the imposed stereotypes do not hinder the determination of the true preferences.

By using the above-mentioned algorithms, the personal characteristics, assessments of personal qualities, interests, inclinations, talents or abilities are compared with the requirements both for professions in general and for the vacancies of particular companies while taking into account their corporate culture. The vocational guidance for specific vacancies of particular companies or for the profession in general is conducted in such a manner.

For example, it turns out that a test subject corresponds to the STEM (science, technology, engineering, mathematics) specialty with 97% probability, to an accountant with 40% probability and to a project manager with only 5%.

According to the test data obtained, some application embodiments involve personal offers to respond to the most suitable vacancies based on profile education. Information about vacancies may be either known from other sources or available only to a limited number of persons who have previously passed the qualification testing.

After all, according to the test results, a person can make a more informed decision about the next career step and any required additional training, preferred products and services. For example, a candidate considered by companies or educational institutions may obtain preferential treatment or receive an individual special offer for products or services if the test results revealed that he is their preferred candidate or client.

**Example 08:** Owing to the lack of detailed data about personal traits and individual abilities of existing and potential clients, future employers, educational institutions, banks, insurance companies and other organizations are forced to focus on the average user metrics. Thus, clients who are potentially more profitable for a company and may generate more income or fewer losses (in case of compensations) can receive product and service offers on the same commercial terms as less profitable clients.

This raises the challenge of more accurate segmentation of clients (up to segments consisting of one client) in order to make more personalized offers of products and services based on additional information.

Let's assume that the number of loan defaults in a single bank remains high. The head of the loan portfolio is exploring ways to reduce the number of bad loans. How is it possible to find out more about a client's loan performance if there is a lack of either publicly available information or the bank's internal information about the client? How is it possible to improve the determination of an individual credit limit or to make more money on reliable solvent borrowers as well as avoid losses stemming from less reliable ones? What are other ways to adjust tariffs or other terms of providing products and services by optimizing the risk/return ratio?

Let's assume that a manager of an insurance company thinks about learning how to identify high-risk clients in order to reduce the number of unprofitable clients. How can they attract good clients who avoid insurance cases with low rates in a highly competitive market, and how can they set prohibitive tariffs for more risky clients or otherwise adjust the tariffs or other terms of providing products and services by optimizing the risk/return ratio? How is it possible to find out more about a client's personal characteristics and assess the probability of loss relating to an insurance contract if there is no previous history of the client?

For example, a bank/insurance company sends a client who wishes to obtain a credit or insure property an individual URL link to the game that identifies a candidate's personal parameters and adds extra parameters that were lacking before to the scoring model of the bank/insurance company. Now the bank/insurance company knows even more about its clients and can make a more informed decision about their degree of risk.

In some embodiments, the analysis of the results is provided immediately, which accelerates the decision-making process. For example, a client is informed about the approved personal loan amount or the cost of insurance (or the approved discount).

As a result, at the very least, the bank/insurance company obtains additional information, such as the performance or propensity for fraud and a borrower's/insured person's degree of risk, which may complement the existing scoring models.

Thus, the bank/insurance company obtains an opportunity to set up a higher individual credit threshold, lower an insurance rate etc. with less risk in order to attract more profitable clients or to set prohibitive barriers for less profitable ones and otherwise adjust the tariffs or other terms of providing products and services by optimizing the risk/return ratio. This means the bank/insurance company may potentially earn more on reliable clients (or other client categories) and experience fewer losses or income loss from less profitable clients (or other client categories) or avoid losses from potential fraudulent activities (including potential ones) while retaining the loyalty of its existing clients with special offers or otherwise increasing its revenue and profits by optimizing the balance of risk and return.

In some embodiments, testing can help to identify clients for whom the price is either less or more important than quality parameters and volume of services as well as identify potential cross-sales opportunities for related products and services, which may generate additional revenue or reduce losses.

**Example 09:** An owner of an electronic store thinks of how to improve its recommendation system so that a client could immediately get the offers he is interested in. How can they find out what type of advertising affects a client most? Does a client prefer text and digital information or images or audio? Which arguments have a greater effect: rational or emotional? What causes a client to doubt and prevents him from making a spontaneous purchase? The recommendation system's quality directly affects the income of the electronic store.

This raises the challenge of improving the recommendation system of an electronic store so that it can identify the needs and interests of a client as soon as possible and display in the search results only the offers he is interested in. Since advertising and recommendations are perceived differently by users, it is necessary to learn about a buyer and about what type of advertising affects him the most: a short or detailed text, a table, an image or a video. It is necessary to determine what affects the spontaneity of his purchase and what causes a buyer's doubts and prevents him from making a spontaneous purchase. Which arguments have a greater effect: rational or emotional?

An electronic store invites a client to click on an individual URL link and take a test in the form of a game by motivating the client with bonuses. The game or test application, in addition to entertaining the client, determines one or more of the desired characteristics of his psychological profile. At the same time, the game or test is arranged so that it is not obvious to a client how the measurement of his skills and abilities is taking place; therefore, the client cannot falsify the result by asking another player to play or take the test instead.

The identified personal parameters of a client add extra input parameters to the recommendation system algorithm that were lacking before. Now the electronic store knows even more about its clients and can offer products and services they are more interested in.

As a result, the owner of an electronic store can generate more sales due to a greater number of spontaneous purchases being made as well as a reduction in the time before purchases are made. It may also increase client loyalty to the service as they avoid unnecessary searches and find what they need with less effort. Additional information on clients' preferences can help the owner to expand the range of goods with those he might not have previously known would be of interest to clients, and he will be able to attract additional traffic to the electronic store. In this connection, the number of clients and sales per client or goods turnover may increase. In some cases, it may help to reduce costs and provide clients with additional benefits through lower prices for all or certain categories of goods and services and even to raise the prices in the case of inelastic demand or when focusing on the premium segment. The additional information obtained about clients may be useful for making a decision on adjusting the marketing positioning of a brand of the company or electronic store and selected goods or categories.

**Example 10:** A customer, an operator or another real-time bidder (Real Time Bidding, RTB) or participant of another platform, online advertising technology, interactive physical advertising (including visual and audio panels and systems) thinks of how to improve the effectiveness of advertising as well as how to decrease advertising costs or increase revenue and profits from the sale of advertising. How can they increase the number of views of advertisements or reactions to advertisements (including clicks on online advertisements on websites, in videos, or in applications or physical or virtual, including built-in, purchases of products and services)?

An electronic store, a physical store, an operator or another participant of a platform, an online advertising technology or interactive physical advertising asks a client to click on an individual URL link and take a test in the form of a game by motivating the client with bonuses or otherwise. The game or test application, in addition to entertaining the client, determines one or more of the desired characteristics of his psychological profile. At the same time, the game or test is arranged so that it is not obvious to the client how the measurement of his skills and abilities is taking place, so the client cannot falsify the results by asking another player to play or take the test instead.

The identified personal parameters of a client add extra input parameters that were lacking before to the algorithm of the interactive advertising system. Now the advertising platform knows even more about the people viewing the advertisements and can offer advertisements of products or services that they have a greater interest in, including varying the way or frequency of the offering, geolocation, advertising content etc.

In some embodiments, such additional information, such as information about personal characteristics, inclinations, preferences and the emotional state of a consumer, along with a known person's geo-position, can help him to make a spontaneous or a considered purchase, including along the way or with a deviation toward the place of provision or consumption of products and services.

As a result, a consumer of advertising receives fewer advertisements that do not meet his interests, location or desired way and frequency of receiving advertisements, and the customer, operator of a platform, online advertising technology, interactive physical advertising will increase the effectiveness of advertising displays, reduce advertising costs and increase the received revenue or profits from the sales of advertising. Some embodiments imply an increase in the number of advertising views or reactions to the advertising.

**Example 11:** An owner of an electronic platform for learning or skills training, as well as other platforms for individual or collaborative learning (with or without a teacher), poses the following question: How can the learning be made more interactive and adaptive? Also, what is the best way to take into account or influence the degree or ability to assimilate particular information in one form or another (in the form of a text, an audio or video presentation or an interaction with a robotic teacher or a live person, including a teacher, mentor, coach and other people taking part in the learning process, such as students or fellow students) when interacting with trainees? How can additional information about the participants of the learning process be obtained, used, transferred and exchanged between computerized systems and the participants of the learning process (including via chat-bots, messengers, video chats and other means of communication, interaction or training).

Students, teachers and other participants of the above-mentioned platform receive an individual URL link or an activation code to at least one mobile game or another task that automatically evaluates one or more parameters: the personal characteristics, personal qualities, interests, inclinations, talents or abilities of the user. Furthermore, this user may be required to take this test repeatedly, with either longer or shorter time intervals. Since emotions (both conscious and subconscious) are also taken into account during game play or task solving, the obtained data relating to test results over time allows for the prediction of the degree of change in the personal characteristics, personal qualities, interests, inclinations, talents or abilities of the user as well as the probability of these changes. In some embodiments, a test may be performed both in an external application and with the functionality built into the platform (including the Learning Management System (LMS) or via chat-bots, messengers, video chats or other means of communication, interaction or training).

The identified personal parameters of students, teachers and other participants of the above-mentioned platform add extra input parameters that were lacking before to the algorithm of the training and the adaptive or interactive platform. Now the platform or its participants know even more about its users or about each other, which allows for recommendations to be made for increasing the effectiveness of their interaction, including within the framework of training.

As a result, an electronic platform for learning, skills training or another platform for individual or collaborative learning (with or without a teacher) is able to provide additional interactivity and adaptability to the learning process, which either directly or indirectly influences the degree, speed or sustainability of information assimilation and the increase in loyalty to particular training products and services. In some embodiments, this allows for the improvement of the match between the teaching style and the preferences of students or regarding the teaching style, which increases the effectiveness of perception, assimilation of information and allows for the provision of feedback and recommendations to the above-mentioned participants, such as how to increase their effectiveness and other useful recommendations. In some embodiments, this allows for the formation of groups with similar psychophysiological and cognitive traits, which contributes to the effectiveness of training, information perception and the overall satisfaction of the participants with the process.

**Example 12:** A creator, an operator or a customer of a Human Machine Interface (HMI) thinks of how to personalize the interaction between a human and a machine either in the form of physical interaction (an android, a robot, a payment terminal etc.) or virtual interaction (a chat-bot, a virtual personal assistant etc.), although both methods could be used as well. How can a perception be created that the machine has empathy, and how can its virtual analogue engender a feeling that the machine recognizes a person's emotions, personal characteristics, personal qualities, interests, inclinations, talents, abilities and more?

A person who wishes to interact with the machine receives an individual URL link or an activation code to at least one mobile game (including one with a virtual or augmented reality interface) or another task that automatically evaluates one or more parameters: the personal characteristics, personal qualities, interests, inclinations, talents or abilities of the user. In some embodiments a test may be performed both in an external application and with the functionality built into the platform (including via the human-machine interface itself or via chat-bots, messengers, video chats or other means of communication or interaction) using a contact (by pressing buttons, touching a screen, moving a manipulator, measuring biological and kinetic parameters through wearable devices) or a non-contact method (nodding or other gestures, eye movements, voice control etc.) as well as other methods that can be used once, repeatedly or continuously.

The identified personal parameters of people interacting with the machine then adds extra input parameters that were lacking before to the algorithm that controls the human-machine interaction. Now the machine knows even more about the people who interact with it, which allows it to make recommendations and to increase the effectiveness of their interactions.

As a result, a machine creates the impression of having empathy, its virtual analogue and/or a feeling that the machine recognizes a person's emotions, personal characteristics, qualities, interests, inclinations, talents, abilities and more. In some embodiments, this leads to overcoming the barriers of interaction via a human-machine interface, increasing a person's satisfaction from the interaction (in terms of loyalty to the interaction, preferred duration, emotional mood etc.) and increasing the effectiveness of the interaction (in terms of the speed of semantic information transmission, the number of errors in recognizing the meaning of what was said etc.).

The present invention was shown and described in a form that is considered to be the most practical and preferred option for implementation. However, within the scope of the invention, it is evident that the necessary differences can still be made and that obvious modifications will be understandable for a person skilled in the art. With regards to the foregoing description, it should be understood that minor changes in the parts of the invention, including changes in its functions and way of working and usage are considered to be obvious for a person skilled in the art, and all equivalent ratios shown in the drawings and described herein are intended to be covered by the present invention.

Therefore, the foregoing is only considered as illustrative of the principles of the invention. Furthermore, since numerous modifications and changes will be readily available to those who are skilled in the art, it is not desirable to limit the invention to the exact construction and operation that has been shown and described. Accordingly, any suitable modifications and equivalents fall within the scope of the claimed protection of the invention.

INFORMATION SOURCES USED

1. Hunter, J. E., & Schmidt, F. L. (1983). Quantifying the effects of psychological interventions on employee job performance and work-force productivity. American Psychologist, 38(4), 473-478.

2. Muchinsky P. M. Validation of intelligence and mechanical aptitude tests in selecting employees for manufacturing jobs // Journal of Business and Psychology. - 1993. - Vol. 7. - No. 4. - pp. 373-382.

3. Finucane M. L., Gullion C. M. Developing a tool for measuring the decision-making competence of older adults // Psychology and aging. - 2010. - Vol. 25. - No. 2. - pp. 271.

4. Phillips P. J. et al. The FERET evaluation methodology for face-recognition algorithms // IEEE Transactions on pattern analysis and machine intelligence. - 2000. - Vol. 22. - No. 10. - pp. 1090-1104.

5. Ekman P., Rosenberg E. L. (ed.). What the face reveals: Basic and applied studies of spontaneous expression using the Facial Action Coding System (FACS). - Oxford University Press, USA, 1997.

6. Crisan L. G. et al. Genetic contributions of the serotonin transporter to social learning of fear and economic decision making // Social cognitive and affective neuroscience. - 2009. - Vol. 4. - No. 4. - pp. 399-408

**Claims**

1. A method for the preparation of recommendations for decision-making based on a computerized assessment of a user's personality and abilities, the method comprising the following steps:

   • granting the user access to at least one computerized task for solving;
   • real-time recognition of the user's emotions while solving at least one task in the presence of at least one video camera;
   • obtaining data about at least one task solved by the user;
   • determining at least one user's ability based both on the data obtained after solving at least one task and on the recognized user's emotions; and
   • providing the user with recommendations for decision-making based on at least one ability determined in the previous step.

2. The method according to claim 1, **characterized in that** the computerized task is a computational task, video interview or game.

3. The method according to claim 1, **characterized in that**: a biometric authentication is performed when a user is granted access to at least one computerized task for solving.

4. The method according to claim 1, **characterized in that** user identification is additionally performed when recognizing a user's emotions.

5. The method according to claim 1, **characterized in that** sensors of wearable devices are used when recognizing a user's emotions.

6. The method according to claim 1, **characterized in that** a user's emotions are recognized based on his speech while taking into account its speed and tone.

7. The method according to claim 1, **characterized in that** a convolutional neural network or a recurrent neural network is used when recognizing a user's emotions.

8. The method according to claim 1, **characterized in that** the data relating to the solution of a task by a user are the user action parameters that are fixed during the solution process.

9. The method according to claim 1, **characterized in that** a user's ability score is determined by presetting the weighting factors to the data relating to the solution of a task by the user as well as the recognized emotions.

10. The method according to claim 1, **characterized in that** a recommendation for a user is a vacancy or a set of vacancies that may be of interest to the user.

```
┌─────────────────────────────────────────┐
│                                           │
│  Granting the user access to at least one │      101
│  computerized task for solution           │
│                                           │
│                                           │
└─────────────────────────────────────────┘
                    │
                    ▼
┌─────────────────────────────────────────┐
│                                           │
│  Real-time recognition of the user's      │
│  emotions while solving at least one task │      102
│  in the presence of at least one video    │
│  camera                                   │
│                                           │
└─────────────────────────────────────────┘
                    │
                    ▼
┌─────────────────────────────────────────┐
│                                           │      103
│  Obtaining data about at least one task   │
│  solved by the user                       │
│                                           │
└─────────────────────────────────────────┘
                    │
                    ▼
┌─────────────────────────────────────────┐
│                                           │
│                                           │
│  Determining at least one ability of the  │
│  user based on both the data obtained     │      104
│  after solving at least one task and on   │
│  the recognized user's emotions           │
│                                           │
│                                           │
└─────────────────────────────────────────┘
                    │
                    ▼
┌─────────────────────────────────────────┐
│                                           │
│  Providing the user with recommendations  │      105
│  for decision making based on at least    │
│  one ability determined in the previous   │
│  step                                     │
│                                           │
└─────────────────────────────────────────┘
```

Fig. 1

Figure 2

Figure 3

406

403

402

404

402

401

402

405

Figure 4

Figure 5

Figure 6

403

404

405

Figure 7

Figure 8

## INTERNATIONAL SEARCH REPORT

| International application No. |
|---|
| PCT/RU 2017/000791 |

| A. CLASSIFICATION OF SUBJECT MATTER |
|---|
| A61B 5/16 (2006.01); A61B 5/02 (2006.01) |
| According to International Patent Classification (IPC) or to both national classification and IPC |

| B. FIELDS SEARCHED |
|---|
| Minimum documentation searched (classification system followed by classification symbols) |
| A61B 5/00-5/16, G06K 9/00, G06Q 10/00-10/10 |
| Documentation searched other than minimum documentation to the extent that such documents are included in the fields searched |
| |
| Electronic data base consulted during the international search (name of data base and, where practicable, search terms used) |
| PatSearch (RUPTO internal), USPTO, PAJ, Esp@cenet, DWPI, EAPATIS, PATENTSCOPE, Information Retrieval System of FIPS |

| C. DOCUMENTS CONSIDERED TO BE RELEVANT |
|---|

| Category* | Citation of document, with indication, where appropriate, of the relevant passages | Relevant to claim No. |
|---|---|---|
| X | CN 104644189 A (LIU ZHENJIANG) 27.05.2015, item 1 of the claims, the abstract, [0004], [0005], [0012], [0013], [0014] | 1-2, 5-6, 8-9 |
| Y | | 3-4, 7, 10 |
| Y | CN 106920074 A (FOSHAN RONGXINTONG ENTERPRISE CONSULTING) 04.07.2017, item 1 of the claims | 3-4, 10 |
| Y | WO 2017/213558 A2 (AVERYANOV V. V. et al) 14.12.2017, p. 7, p. 18-24 | 7 |
| A | RU 168332 U1 (AVERYANOV VITALY VITALEVICH et al.) 30.01.2017 | 1-10 |

| ☐ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 06 August 2018 (06.08.2018) | 09 August 2018 (09.08.2018) |

| Name and mailing address of the ISA/ RU | Authorized officer |
|---|---|
| | |
| Facsimile No. | Telephone No. |

Form PCT/ISA/210 (second sheet) (July 1998)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6275812 B1

**Non-patent literature cited in the description**

- **HUNTER, J. E. ; SCHMIDT, F. L.** Quantifying the effects of psychological interventions on employee job performance and work-force productivity. *American Psychologist,* 1983, vol. 38 (4), 473-478
- **MUCHINSKY P. M.** Validation of intelligence and mechanical aptitude tests in selecting employees for manufacturing jobs. *Journal of Business and Psychology,* 1993, vol. 7 (4), 373-382
- **FINUCANE M. L. ; GULLION C. M.** Developing a tool for measuring the decision-making competence of older adults. *Psychology and aging,* 2010, vol. 25 (2), 271

- **PHILLIPS P. J. et al.** The FERET evaluation methodology for face-recognition algorithms. *IEEE Transactions on pattern analysis and machine intelligence,* 2000, vol. 22 (10), 1090-1104
- What the face reveals: Basic and applied studies of spontaneous expression using the Facial Action Coding System (FACS). Oxford University Press, 1997
- **CRISAN L. G. et al.** Genetic contributions of the serotonin transporter to social learning of fear and economic decision making. *Social cognitive and affective neuroscience,* 2009, vol. 4 (4), 399-408